# EUROPÄISCHE PATENTSCHRIFT

(11) **EP 1 162 187 B1**
(45) Veröffentlichungstag und Bekanntmachung des Hinweises auf die Patenterteilung: **07.12.2005**
(21) Anmeldenummer: 01112681.0
(22) Anmeldetag: 25.05.2001
(51) Int. Cl.: C07C 29/70, C07C 31/125

(54) **Verfahren zur Herstellung von alkoholischen Lösungen von Alkalialkoholaten**
Process for the preparation of alcoholic solutions of alkali metal alcoholates
Procédé de préparation de solutions alcooliques d'alcoolats de métaux alcalins

(30) Priorität: 09.06.2000 DE 10028754
(43) Veröffentlichungstag der Anmeldung: 12.12.2001
(73) Patentinhaber: Clariant Finance (BVI) Limited, Road Town, Tortola (VG)
(72) Erfinder: Nickel, Uwe, Dr., 61352 Bad Homburg (DE); Unverdorben, Leonhard, Dr, 61130 Nidderau (DE); Weber, Joachim, Dr., 65929 Frankfurt am Main (DE); Dietz, Erwin, Dr., 61462 Königstein (DE); Patzlaff, Jürgen, Dr., 64380 Rossdorf (DE)
(74) Vertreter: Hütter, Klaus

(56) Entgegenhaltungen:
- EP-A- 0 749 947
- DE-A- 2 612 642
- DE-A- 3 926 466
- DE-B- 2 333 634

## Beschreibung

Die vorliegende Erfindung beschreibt ein Verfahren zur Herstellung von alkoholischen Lösungen von Alkalialkoholaten in Mikroreaktoren.

Alkalialkoholate sind als Zwischenprodukte, Reaktionspartner und Katalysatoren bei der Synthese vieler organischer Verbindungen von großer Bedeutung. Zu ihrer Herstellung ist eine Reihe verschiedener Verfahren bekannt.

Die Alkalialkoholate der tertiären Alkohole haben als Katalysatoren und Kondensationsmittel wachsendes Interesse gefunden, da die zugehörigen Alkohole sterisch gehindert sind, eine geringe Acidität besitzen und damit zum einen stärkere Protonen-Akzeptoren sind und zum anderen wesentlich weniger zu Nebenreaktionen neigen als primäre oder sekundäre Alkohole. In vielen Fällen werden die Alkoholate in Form ihrer Lösungen im entsprechenden Alkohol eingesetzt. Der Alkohol kann gleichzeitig als Lösemittel bei der gewünschten Reaktion dienen, so dass die Zahl der eingesetzten Lösemittel reduziert werden kann und so kostengünstige Verfahren mit geringerem apparativen Aufwand möglich sind. Außerdem ist die Handhabung der Alkoholate in Form ihrer Lösungen technisch wesentlich unproblematischer als in fester Form.

Eine der Verfahrensweisen zur Herstellung von Alkoholaten besteht darin, dass man das freie Alkalimetall mit dem Alkohol direkt umsetzt. Es ist z.B. aus der DE-A-26 12 642 bekannt, dass sich mit zunehmender Kettenlänge des Alkohols die Umsetzung verlangsamt, ebenso mit zunehmendem Verzweigungsgrad: primäre Alkohole reagieren am schnellsten, tertiäre am langsamsten.

Aus der Literatur sind verschiedene spezielle Verfahren zur Herstellung von Alkoholaten bekannt.

Die DE-A-23 33 634, DE-A-26 12 642 und EP-A-0 749 947 offenbaren Verfahren zur Herstellung von alkoholfreien Alkalialkoholaten in einem inerten Lösemittel, bei denen die Umsetzung des Alkalimetalls mit dem Alkohol Batch-weise durchgeführt wird. Bei der DE-A-23 33 634 wird erhöhter Druck und Temperatur zur Reaktionsbeschleunigung eingesetzt, bei der DE-A-26 12 642 und EP-A-0 749 947 wird das Natriummetall in fein verteilter Form eingesetzt. Alle diese Verfahren haben jedoch den Nachteil, dass ein zusätzliches inertes Lösemittel eingesetzt wird. Um eine alkoholische Lösung des Alkalialkoholats zu erhalten, muss dieses inerte Lösemittel erst wieder vollständig abgetrennt werden und das erhaltene, feste Alkoholat im Alkohol wieder gelöst werden. Dadurch werden zusätzliche Kosten verursacht, ein größerer apparativer Aufwand ist erforderlich und die Prozesszeiten verlängern sich.

Die EP-A-0 192 608 offenbart ein Batch-Verfahren zur Herstellung von alkoholischen Lösungen von Alkali-tert.-alkoholaten durch Umsetzen eines Alkalimetalls mit einem tertiären Alkohol, indem der heiße Alkohol zum geschmolzenen Alkalimetall unter Rühren mit einem Anker- oder Flügelrührer zugegeben wird. Auch hier betragen die Reaktionszeiten Stunden. Außerdem können Alkohole mit Siedepunkten unterhalb des Schmelzpunkts des eingesetzten Alkalimetalls nicht mit ausreichender Reaktionsgeschwindigkeit umgesetzt werden, da das feste Alkalimetall im Lösemittel nicht ausreichend dispergiert werden kann.

Die WO 99/65849 offenbart ein Batch-Verfahren zur Herstellung von festen Alkalialkoholaten in Gegenwart eines Katalysators, der jedoch nicht mehr abgetrennt wird.

Ein gemeinsames Merkmal dieser Verfahren sind die relativ große Mengen Alkalimetall, die auf einmal eingesetzt werden müssen und die mit Alkohol in Berührung gebracht werden, so dass auch größere Mengen Wasserstoff in kurzer Zeit entstehen. Dies führt zu hohen sicherheitstechnischen Anforderungen in der großtechnischen Produktion. Um wirtschaftlich sinnvolle Reaktionszeiten zu erzielen, ist die Feinverteilung des Alkalimetalls entscheidend, diese wird meist durch intensives mechanisches Dispergieren erzielt. Auch der scale-up von Verfahren vom Labormaßstab in den großtechnischen Maßstab ist bei Batch-Verfahren aufwendig und kann Schwierigkeiten bereiten, da beispielsweise Kessel- und Rührergeometrien oder Wärmeübergänge großen Einfluss auf beispielsweise Reaktionszeiten und Umsätze haben.

Der vorliegenden Erfindung lag die Aufgabe zugrunde, ein Verfahren zur kostengünstigen, technisch zuverlässigen und schnellen Herstellung alkoholischer Lösungen von Alkalialkoholaten zu finden, das auch im Falle der höheren und verzweigten Alkohole zu wirtschaftlichen Reaktionszeiten und produktionstechnisch befriedigenden Verfahren führt, wobei der scale-up einfach zu bewerkstelligen und das Sicherheitsrisiko des Verfahrens gering gehalten werden kann, z.B. durch Minimierung der zugegebenen Alkalimetallmenge pro Reaktorvolumen.

Es ist bekannt, bestimmte chemische Umsetzungen in Mikroreaktoren durchzuführen. Mikroreaktoren sind aus Stapeln von strukturierten Platten aufgebaut und in der DE 39 26 466 C2, US-A-5,534,328 und US-A-5,811,062 beschrieben.

Es wurde nun gefunden, dass sich Mikroreaktoren überraschenderweise zur Herstellung von alkoholischen Lösungen von Alkalialkoholaten eignen.

Die verwendete Bezeichnung Mikroreaktor steht dabei stellvertretend für Mikro- und Minireaktoren, die sich nur aufgrund der Dimensionen und Aufbau der Reaktionskanalstrukturen unterscheiden.
Beispielsweise können Mikroreaktoren, wie sie aus den angeführten Schriften oder aus Veröffentlichungen des Instituts für Mikrotechnik Mainz GmbH, Deutschland, bekannt sind, oder auch kommerziell erhältliche Mikroreaktoren, wie beispielsweise der auf Cytos™ basierende Selecto™ der Firma Cellular Process Chemistry GmbH, Frankfurt/Main, eingesetzt werden.

Gegenstand der Erfindung ist ein Verfahren zur Herstellung von alkoholischen Lösungen von Alkalialkoholaten, dadurch gekennzeichnet, dass ein Alkalimetall mit einem Alkohol in einem Mikroreaktor umgesetzt wird.

Bei dem erfindungsgemäßen Verfahren werden das Alkalimetall und der Alkohol in flüssiger oder geschmolzener Form dem Mikroreaktor zugeführt, im Innern des Mikroreaktors kontinuierlich miteinander vermischt und zur Umsetzung gebracht. An Stelle des reinen Alkohols kann auch eine alkoholische Lösung eines Alkalialkoholats niedriger Konzentration verwendet und durch die Umsetzung mit dem Alkalimetall im Mikroreaktor konzentriert werden. Auch kann die gebildete Alkoholatlösung im Kreis gefahren werden. Im folgenden werden die beiden zugeführten Umsetzungspartner, das Alkalimetall und der Alkohol bzw. die Alkoholatlösung, Materialströme genannt.

Der entstandene Wasserstoff wird zweckmäßigerweise an Entgasungszonen abgeleitet.

Es können auch die im herkömmlichen Verfahren verwendeten Hilfsmittel im erfindungsgemäßen Verfahren eingesetzt werden.

Als Alkalimetalle werden vorzugsweise Lithium, Natrium, Kalium oder eine Lithium-, Natrium- oder Kalium-enthaltende Legierung, bevorzugt Natrium oder Kalium, insbesondere Natrium, eingesetzt.

Das erfindungsgemäße Verfahren wird zur Herstellung von alkoholischen Lösungen von Alkalialkoholaten von Alkoholen mit 1 bis 30 C-Atomen, bevorzugt mit 3 bis 30 C-Atomen, besonders bevorzugt für sekundäre, tertiäre und verzweigte Alkohole mit 4 bis 30 C-Atomen, insbesondere für sek.-Butanol, tert.-Butanol, tert.-Amylalkohol, 3,7-Dimethyl-1,6-octadien-3-ol (Linalool), 3,7,11-Trimethyl-3,6,10-dodecatrien-3-ol, 3,7,11,15-Tetramethyl-1-hexadecen-3-ol und Tetrahydrolinalool, angewendet. Eine weitere bevorzugte Anwendung ist die Herstellung von alkoholischen Lösungen von Alkalialkoholaten von längerkettigen Alkoholen, wie beispielsweise Stearylalkohol, oder von mehrwertigen Alkoholen.

Der Alkohol wird in einem solchen Überschuss zum eingesetzten Alkalimetall eingesetzt, dass das gebildete Alkoholat stets in Lösung bleibt bzw. stets eine feststofffreie Schmelze vorliegt.

Während der Reaktion wird die Temperatur zweckmäßigerweise oberhalb des Schmelzpunkts des Alkalimetalls, des Alkohols und oberhalb des Schmelzpunkts der sich bildenden alkoholischen Lösung des Alkalialkoholats gehalten. Die Temperaturen können zwischen 25°C und 250°C, bevorzugt zwischen 25°C und 200°C, insbesondere zwischen 25°C und 150°C, liegen. Die Reaktion kann auch vorteilhaft unter erhöhtem Druck durchgeführt werden, beispielsweise bei Alkoholen, deren Siedepunkt bei Normaldruck unterhalb oder nur wenig oberhalb des Schmelzpunkts des Alkalimetalls liegt, um bei Temperaturen oberhalb des Schmelzpunktes des Alkalimetalls die Umsetzung durchführen zu können und nicht nahe des Siedepunkts des Alkohols arbeiten zu müssen. Die Drücke können zwischen Normaldruck und 100 bar Überdruck, bevorzugt zwischen Normaldruck und 50 bar, insbesondere zwischen Normaldruck und 25 bar, liegen.

Die Konzentration der hergestellten Alkoholatlösung ist zweckmäßigerweise bis zu 70 Gew.-%, bevorzugt 5 bis 60 Gew.-%, insbesondere 10 bis 50 Gew.-%.

Ein Mikroreaktor ist aus mehreren aufeinandergestapelten und miteinander verbundenen Plättchen aufgebaut, auf deren Oberflächen sich mikromechanisch erzeugte Strukturen befinden, die in ihrem Zusammenwirken Reaktionsräume bilden, um chemische Reaktionen auszuführen. Es ist wenigstens ein durch das System hindurchführender Kanal enthalten, der mit dem Einlass und dem Auslass verbunden ist.
Die Flussraten der Materialströme sind apparativ limitiert, beispielsweise durch die sich je nach geometrischer Auslegung des Mikroreaktors einstellenden Drücke. Es ist wünschenswert, dass die Reaktion im Mikroreaktor vollständig abläuft, es können sich aber auch Vermischungszonen in Form von Mikromischem und/oder Verweilzonen anschließen. Ebenso können die Materialströme wiederholt an mehreren, hintereinander angeordneten Stellen zugeführt werden.

Die Flussraten sind zweckmäßigerweise zwischen 0,05 ml/min und 5 l/min, bevorzugt zwischen 0,05 ml/min und 500 ml/min, besonders bevorzugt zwischen 0,05 ml/min und 250 ml/min, und insbesondere zwischen 0,1 ml/min und 100 ml/min. Die Flussraten der beiden Materialströme können verschieden sein.

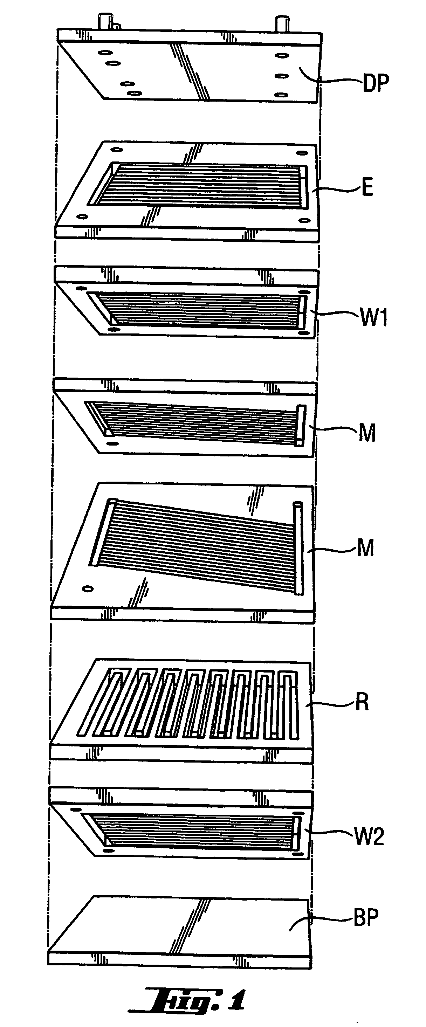

Beispielhaft wird in Figur 1 ein für die Teilschritte der Herstellung von alkoholischen Lösungen von Alkalialkoholaten einsetzbarer Mikroreaktor beschrieben.
Das vorliegende Mikroreaktionssystem ist in diesem Fall ein aus sechs, aufeinander gestapelten und miteinander verbundenen mikrostrukturierten Blechlagen und je einer Deckelplatte (DP) und Bodenplatte (BP) aufgebautes verfahrenstechnisches Modul, das durch den Zusammenbau unter Druck gehalten oder fest verbunden wird, um Abdichtflächen zwischen den Platten zusammenzupressen.
Das vorliegende Mikroreaktionssystem beinhaltet zwei Wärmetauscher für Kühl- und/oder Heizmedium, eine Mischzone für die Vermischung der Reaktanden sowie eine kurze Verweilstrecke.
Mit Hilfe des Wärmetauschers (W1) werden die in Platte (E) getrennt einströmenden Materialströme vortemperiert. In den Platten (M), die ein gemeinsames Volumen bilden, findet dann die Vermischung der Materialströme statt. In der Verweilzone (R) wird das Reaktionsgemisch mit Hilfe des Wärmetauschers (W2) auf die vorstehend genannten Reaktionstemperaturen gebracht, so dass die jeweilige Reaktion stattfinden kann.

Das Mikroreaktionssystem wird vorzugsweise kontinuierlich betrieben, wobei sich die jeweils miteinander zur Vermischung gebrachten Materialmengen im Mikro- (µl) bis Milliliter (ml) -Bereich bewegen.

Entscheidend für die Herstellungsschritte bei alkoholischen Lösungen von Alkalialkoholaten in einem Mikroreaktionssystem sind die Dimensionen der mikrostrukturierten Bereiche innerhalb eines Reaktors. Durch entsprechende geometrische Gestaltung wird dafür gesorgt, dass keine Totzonen, wie z.B. Sackgassen oder scharfe Ecken, vorhanden sind. Bevorzugt sind daher kontinuierliche Bahnen mit abgerundeten Ecken. Die Strukturen müssen klein genug sein, um die immanenten Vorteile der Mikroreaktionstechnik auszunutzen, nämlich hervorragende Wärmekontrolle, laminare Schichtenströmung, diffusives Mischen und geringes internes Volumen.

Die lichte Weite der materialführenden Kanäle beträgt zweckmäßigerweise 0,1 bis 10000 µm, vorzugsweise 1 bis 2000 µm, besonders bevorzugt 1 bis 800 µm, insbesondere 1 bis 100 µm.

Die lichte Weite der Wärmetauscherkanäle richtet sich in erster Linie nach der lichten Weite der flüssigkeits- oder suspensionsführenden Kanäle und ist zweckmäßigerweise kleiner oder gleich 10000 µm, vorzugsweise kleiner oder gleich 2000 µm, insbesondere kleiner oder gleich 800 µm. Die Untergrenze der lichten Weite der Wärmetauscherkanäle ist unkritisch und wird allenfalls durch den Druckanstieg der zu pumpenden Wärmetauscherflüssigkeit und von der Notwendigkeit der optimalen Wärmezufuhr oder -abfuhr begrenzt.

Die Dimensionen eines bevorzugt verwendeten, in Fig.1 beispielhaft dargestellten Mikroreaktionssystems sind:

| | |
|---|---|
| Wärmetauscherstrukturen | Kanalbreite ∼600 µm |
| | Kanalhöhe ∼250 µm |
| | |
| Mischer | Kanalbreite ∼600 µm |
| | Kanalhöhe ∼500 µm |

Im beispielhaft beschriebenen Mikroreaktor-Typ werden die sechs aufeinanderliegenden und dicht miteinander verbundenen Blechlagen vorzugsweise von oben mit allen Wärmetauscherfluiden und Reaktanden beschickt. Die Abfuhr des Produktes und der Wärmertauscherfluide erfolgt vorzugsweise ebenfalls nach oben. Die Kontrolle der benötigten Flüsse wird vorzugsweise über Präzisionskolbenpumpen und einer computergesteuerten Regelung vorgenommen. Die Reaktionstemperatur wird über integrierte Sensoren überwacht und mit Hilfe der Regelung und eines Thermostaten/Cryostaten überwacht und gesteuert.

Das hier dargestellte System ist aus Edelstahl gefertigt; andere Materialien wie zum Beispiel Glas, Keramik, Silizium, Kunststoffe oder andere Metalle sind ebenso einsetzbar.

Es war überraschend und nicht vorhersehbar, dass die Herstellung von alkoholischen Lösungen von Alkalialkoholaten in dieser technisch einfachen und zuverlässigen Weise möglich ist, da nicht vorhersehbar war, dass die Reaktion ohne mechanische Durchmischung durchgeführt werden kann, d.h. dass die im Mikroreaktor stattfindende Durchmischung ausreicht.

Das erfindungsgemäße Verfahren kommt ohne den Einsatz weiterer Lösungs- oder Hilfsmittel aus. Dadurch entfällt ein Wiederaufarbeiten beispielsweise eines zweiten Lösemittels. Es ist auch nicht notwendig, die Dispergierung und die Durchmischung durch Zugabe einer oberflächenaktiven Substanz zu verbessern, ebenso ist der Einsatz eines Katalysator nicht erforderlich. Ein weiterer Vorteil ist die relativ niedrige Reaktionstemperatur; bereits bei Temperaturen knapp oberhalb des Schmelzpunkts des Alkalimetalls werden wirtschaftliche und produktionstechnisch befriedigende Durchsätze ermöglicht. Bei niedrigen Temperaturen können auch unerwünschte Nebenreaktionen minimiert werden.

Die erfindungsgemäß hergestellten alkoholischen Lösungen von Alkalialkoholaten können direkt für alle Synthesen, bei welchen alkoholische Lösungen von Alkoholaten, insbesondere von sekundären und tertiären Alkoholaten, benötigt werden, eingesetzt werden.

In den nachstehenden Beispielen beziehen sich Prozentangaben auf Gewichtsprozente.

### Beispiel 1

518 g tert.-Amylalkohol (Siedepunkt bei 2,5 bar: 129°C) werden in einem Vorlagebehälter vorgelegt und bei 120°C im Kreis mit einer Flussrate von 30 ml/min durch den Mikroreaktor gepumpt. Dann werden kontinuierlich 34,5 g Natrium mit einer Temperatur von 103°C und mit einer Flussrate von 0,2 ml/min in den Mikroreaktor zugepumpt. Das druckfest geschlossene Vorlagegefäß ist mit einem Kühler versehen, der nach außen mit einem Überströmventil verschlossen ist. Der bei der Reaktion entstehende Wasserstoff wird über dieses Ventil, das bei einem Druck von mehr als 2,5 bar öffnet, kontrolliert abgeführt. Das Vorratsgefäß, der Reaktor, die Rohrleitungen und die Pumpenköpfe werden auf 120°C temperiert. Nach Beendigung des Zudosierens des Natriums ist keine Wasserstoffentwicklung mehr feststellbar, es hat sich eine 30 %ige Lösung von Natrium-tert.-Amylat in tert.-Amylalkohol gebildet.

## Patentansprüche

1. Verfahren zur Herstellung von alkoholischen Lösungen von Alkalialkoholaten, **dadurch gekennzeichnet, dass** ein Alkalimetall mit einem Alkohol in einem Mikroreaktor umgesetzt wird.

2. Verfahren nach Anspruch 1, **dadurch gekennzeichnet, dass** das Alkalimetall und der Alkohol in geschmolzener oder flüssiger Form dem Mikroreaktor zugeführt, im Innern des Mikroreaktors kontinuierlich miteinander vermischt und zur Umsetzung gebracht werden.

3. Verfahren nach Anspruch 1 oder 2, **dadurch gekennzeichnet, dass** das Alkalimetall Lithium, Natrium, Kalium oder eine Lithium-, Natrium- oder Kalium-enthaltende Legierung ist.

4. Verfahren nach einem oder mehreren der Ansprüche 1 bis 3, **dadurch gekennzeichnet, dass** der Alkohol ein C₁-C₃₀-Alkohol, vorzugsweise ein C₃-C₃₀-Alkohol, insbesondere ein C₄-C₃₀-Alkohol, ist.

5. Verfahren nach mindestens einem der Ansprüche 1 bis 4, **dadurch gekennzeichnet, dass** der Alkohol sek.-Butanol, tert.-Butanol, tert.-Amylalkohol, 3,7-Dimethyl-1,6-octadien-3-ol, 3,7,11-Trimethyl-3,6,10-dodecatrien-3-ol, 3,7,11,15-Tetramethyl-1-hexadecen-3-ol oder Tetrahydrolinalool ist.

6. Verfahren nach mindestens einem der Ansprüche 1 bis 5, **dadurch gekennzeichnet, dass** die Umsetzung oberhalb des Schmelzpunkts des Alkalimetalls, des Alkohols und der sich bildenden Lösung des Alkalialkoholats stattfindet.

7. Verfahren nach mindestens einem der Ansprüche 1 bis 6, **dadurch gekennzeichnet, dass** die Umsetzung zwischen 25 und 250°C, bevorzugt zwischen 25 und 200°C, erfolgt.

8. Verfahren nach mindestens einem der Ansprüche 1 bis 7, **dadurch gekennzeichnet, dass** die eingeleiteten Flüssigkeiten im Innern des Mikroreaktors mit Hilfe eines oder mehrerer Wärmetauscher auf Reaktionstemperatur gebracht und gehalten werden.

9. Verfahren nach mindestens einem der Ansprüche 1 bis 8, **dadurch gekennzeichnet, dass** die Flüssigkeiten in einer kontinuierlichen Bahn mit abgerundeten Ecken durch den Mikroreaktor geleitet werden.

10. Verfahren nach mindestens einem der Ansprüche 1 bis 9, **dadurch gekennzeichnet, dass** die Konzentrationen, Flussraten und Temperaturen über im Mikroreaktor integrierte Sensoren und Regelkreise erfasst und kontrolliert werden.

## Claims

1. A process for preparing an alcoholic solution of an alkali metal alkoxide, which comprises reacting an alkali metal with an alcohol in a microreactor.

2. The process of claim 1, wherein said alkali metal and said alcohol are fed to said microreactor in molten or liquid form and are continuously mixed with each other and reacted in said microreactor.

3. The process of claim 1 or 2, wherein said alkali metal is lithium, sodium, potassium or a lithium, sodium or potassium alloy.

4. The process of one or more of claims 1 to 3, wherein said alcohol is a C₁-C₃₀-alcohol, preferably a C₃-C₃₀-alcohol, especially a C₄-C₃₀-alcohol.

5. The process of at least one of claims 1 to 4, wherein said alcohol is sec-butanol, tert-butanol, tert-amyl alcohol, 3,7-dimethyl-1,6-octadien-3-ol, 3,7,11-trimethyl-3,6,10-dodecatrien-3-ol, 3,7,11,15-tetramethyl-1-hexadecen-3-ol or tetrahydrolinalool.

6. The process of at least one of claims 1 to 5, wherein the reaction takes place above the melting point of said alkali metal, of said alcohol and of the resultant solution of said alkali metal alkoxide.

7. The process of at least one of claims 1 to 6, wherein said reaction is effected between 25 and 250°C, preferably between 25 and 200°C.

8. The process of at least one of claims 1 to 7, wherein the introduced liquids are brought to and held at the reaction temperature in said microreactor by means of one or more heat exchangers.

9. The process of at least one of claims 1 to 8, wherein said liquids pass through said microreactor in a continuous path with rounded corners.

10. The process of at least one of claims 1 to 9, wherein the concentration, flow rates and temperatures are captured and controlled via sensors and control circuits integrated in said microoreactor.

## Revendications

1. Procédé de préparation de solutions alcooliques d'alcoolats de métaux alcalins, **caractérisé en ce que** l'on fait réagir un métal alcalin avec un alcool dans un microréacteur.

2. Procédé selon la revendication 1, **caractérisé en ce que** l'on introduit le métal alcalin et l'alcool sous forme fondue ou liquide dans le microréacteur, on les mélange en continu à l'intérieur du réacteur et on les amène à réagir.

3. Procédé selon la revendication 1 ou 2,
**caractérisée en ce que** le métal alcalin est le lithium, le sodium, le potassium ou un alliage contenant du lithium, du sodium ou du potassium.

4. Procédé selon une ou plusieurs des revendications 1 à 3, **caractérisé en ce que** l'alcool est un alcool en C₁ à C₃₀, de préférence un alcool en C₃ à C₃₀, en particulier un alcool en C₄ à C₃₀.

5. Procédé selon au moins une des revendications 1 à 4, **caractérisé en ce que** l'alcool est le sec.-butanol, le tert.-butanol, le tert.-amylalcool, le 3,7-diméthyl-1,6-octadién-3-ol, le 3,7,11-triméthyl-3,6,10-dodécatrién-3-ol, le 3,7,11,15-tétraméthyl-1-hexadécén-3-ol ou le tétrahydrolinalool.

6. Procédé selon au moins une des revendications 1 à 5, **caractérisé en ce que** la réaction s'effectue au-dessus du point de fusion du métal alcalin, de l'alcool et de la solution de l'alcoolat de métal alcalin se formant.

7. Procédé selon au moins une des revendications 1 à 6, **caractérisé en ce que** l'on effectue la réaction entre 25 et 250 °C, de préférence entre 25 et 200 °C.

8. Procédé selon au moins une des revendications 1 à 7, **caractérisé en ce que** l'on amène et on maintient à la température de réaction les liquides introduits à l'intérieur du microréacteur à l'aide d'un ou de plusieurs échangeurs de chaleur.

9. Procédé selon au moins une des revendications 1 à 8, **caractérisé en ce que** les liquides traversent le microréacteur en un trajet continu ayant des angles arrondis.

10. Procédé selon au moins une des revendications 1 à 9, **caractérisé en ce que** les concentrations, les débits et les températures sont mesurés et régulés au moyen de capteurs et de circuits de régulation intégrés au microréacteur.
